# EUROPEAN PATENT SPECIFICATION

(11) **EP 2 925 329 B1**
(45) Date of publication and mention of the grant of the patent: **06.01.2021**
(21) Application number: 13859049.2
(22) Date of filing: 26.11.2013
(51) Int. Cl.: A61K 35/17, A61K 35/28, A61K 31/66, A61K 35/44, A61P 35/00

(54) **USE OF POST-TRANSPLANT CYCLOPHOSPHAMIDE TREATED ALLOGENEIC MARROW INFILTRATING LYMPHOCYTES TO AUGMENT ANTI-TUMOR IMMUNITY**
VERWENDUNG VON AUF TRANSPLANTATIONEN FOLGENDEN CYCLOPHOSPHAMIDBEHANDELTEN ALLOGENEN KNOCHENMARKSINFILTRIERENDEN LYMPHOZYTEN ZUR STEIGERUNG DER ANTITUMORIMMUNITÄT
UTILISATION DE LYMPHOCYTES INFILTRANT LA MOELLE ALLOGÉNIQUES TRAITÉS PAR CYCLOPHOSPHAMIDE POST-GREFFE POUR AUGMENTER L'IMMUNITÉ ANTI-TUMORALE

(30) Priority: 27.11.2012 US 201261730239 P
(43) Date of publication of application: 07.10.2015
(73) Proprietor: Borrello, Ivan Marques, Baltimore MD 21231 (US); Noonan, Kimberly Ann, Baltimore MD 21209 (US); Luznik, Leonido, Baltimore MD 21210 (US)
(72) Inventor: Borrello, Ivan Marques, Baltimore MD 21231 (US); Noonan, Kimberly Ann, Baltimore MD 21209 (US); Luznik, Leonido, Baltimore MD 21210 (US)
(74) Representative: Stephens, Clare Jean
(86) International application number: PCT/US2013/071975
(87) International publication number: WO 2014/085437

(56) References cited:
- WO-A2-2010/062742
- GIRALT S ET AL: "CD8-DEPLETED DONOR LYMPHOCYTE INFUSION AS TREATMENT FOR RELAPSED CHRONIC MYELOGENOUS LEUKEMIA AFTER ALLOGENEIC BONE MARROW TRANSPLANTATION", BLOOD, AMERICAN SOCIETY OF HEMATOLOGY, US, vol. 86, no. 11, 1 December 1995 (1995-12-01), pages 4337-4343, XP000601434, ISSN: 0006-4971
- ACKERSTEIN A ET AL: "ADOPTIVE TRANSFER OF ALLOGENEIC RLL-2 ACTIVATED DONOR LYMPHOCYTES (ADL) AND RECOMBINANT INTERLEUKIN-2 (RLL-2): AN APPROACH FOR CELL THERAPY IN RELAPSING PATIENTS FOLLOWING ALLOGENEIC BONE MARROW TRANSPLANTATION", BLOOD, AMERICAN SOCIETY OF HEMATOLOGY, US, vol. 86, no. 10, SUPPL. 01, 15 November 1995 (1995-11-15), page 974A, XP002012247, ISSN: 0006-4971
- PETRA HOFFMANN ET AL: "Donor-type CD4 + CD25 + Regulatory T Cells Suppress Lethal Acute Graft-Versus-Host Disease after Allogeneic Bone Marrow Transplantation", THE JOURNAL OF EXPERIMENTAL MEDICINE, vol. 131, no. 3, 5 August 2002 (2002-08-05), pages 2635-399, XP055284140, US ISSN: 0022-1007, DOI: 10.4049/jimmunol.164.1.183
- ANNE-MARIE RASMUSSEN ET AL: "Ex vivo expansion protocol for human tumor specific T cells for adoptive T cell therapy", JOURNAL OF IMMUNOLOGICAL METHODS., vol. 355, no. 1-2, 1 April 2010 (2010-04-01), pages 52-60, XP055240772, NL ISSN: 0022-1759, DOI: 10.1016/j.jim.2010.02.004
- YEE CASSIAN: "Adoptive T-cell therapy of cancer", HEMATOLOGY - ONCOLOGY CLINICS OF NORTH AMERICA, W.B. SAUNDERS, US, vol. 20, no. 3, 1 June 2006 (2006-06-01), pages 711-733, XP008093818, ISSN: 0889-8588, DOI: 10.1016/J.HOC.2006.02.008
- JERAS MATJAZ ET AL: "Induction/Engineering, Detection, Selection, and Expansion of Clinical-Grade Human Antigen-Specific CD8(+) Cytotoxic T Cell Clones for Adoptive Immunotherapy", JOURNAL OF BIOMEDICINE & BIOTECHNOLOGY, 2010, XP002759570, ISSN: 1110-7243
- GEORGIA B. VOGELSANG ET AL: "Pathogenesis and Treatment of Graft-Versus-Host Disease After Bone Marrow Transplant", ANNUAL REVIEW OF MEDICINE : SELECTED TOPICS IN THE CLINICALSCIENCES, vol. 54, no. 1, 1 February 2003 (2003-02-01), pages 29-52, XP055284141, US ISSN: 0066-4219, DOI: 10.1146/annurev.med.54.101601.152339

## Description

### FIELD OF THE INVENTION

The present invention relates to the field of cancer therapy. More specifically, the present invention provides compositions for use in augmenting anti-tumor immunity. The invention is defined by the appended claims.

### BACKGROUND OF THE INVENTION

The use of post-transplant cyclophosphamide administered in the allogeneic bone marrow/stem cell transplant setting has significantly reduced the incidence of graft vs. host disease (GVHD) enabling patients with HLA-identical transplants to often require no additional immune suppression and has also enabled transplantation of non- HLA compatible patients. However, disease relapse remains a major obstacle with poor outcomes. Standard treatment would be the use of donor lymphocyte infusions which is plagued by a significant increase in GVHD with minimal tumor specificity.

WO 2010/062742 A2 (Univ. Johns Hopkins et al.) reports "Methods for Preparation and Use of Marrow Infiltrating Lymphocytes (MILS). Giralt et al, reports "CD8-Depleted Donor Lymphocyte Infusion as Treatment for Relapsed Chronic Myelogenous Leukemia After Allogeneic Bone Marrow Transplantation" (Blood, American Society of Hematology, US, (19951201), Vol. 86, No. 11, Pages 4337 - 4343). Ackerstein et al, reports "Adoptive Transfer of Allogeneic rLL-2 Activated Donor Lymphocytes (ADL) and Recombinant Interleukin-2 (rIL-2): An Approach for Cell Therapy in Relapsing Patients Following Allogeneic Bone Marrow Transplantation" (Blood, American Society of Hematology, US, (19951115), Vol. 86, No. 10, Suppl. 01, Page 974A). Hoffmann et al, reports "Donor-type CD4 + CD25 + Regulatory T Cells Suppress Lethal Acute Graft-Versus-Host Disease After Allogeneic Bone Marrow Transplantation" (The Journal of Experimental Medicine, US, (20020805), Vol. 196, No. 3, Pages 389 - 399). Rasmussen et al, reports "Ex Vivo Expansion Protocol for Human Tumor Specific T Cells For Adoptive T Cell Therapy" (Journal of Immunological Methods., NL, (20100401), Vol. 355, No. 1-2, Pages 52 - 60). Yee, C., reports "Adoptive T-Cell Therapy of Cancer" (Hematology/Oncology Clinics of North America, US, (20060601), Vol. 20, No. 3, Pages 711 - 733). Jeraz et al, reports "Induction/Engineering, Detection, Selection, and Expansion of Clinical-Grade Human Antigen-Specific CD8(+) Cytotoxic T Cell Clones for Adoptive Immunotherapy" (Journal of Biomedicine & Biotechnology, (2010), Article ID 705215, 15 pages).

### SUMMARY OF THE INVENTION

The present invention provides activated marrow infiltrating lymphocytes (MILs) for use in a method for treating or preventing cancer relapse in a subject who has received a post-stem cell transplant cyclophosphamide treatment, the method comprising the step of administering the activated MILs to the subject, wherein the activated MILs are derived from a bone marrow sample taken from the subject following the post-stem cell transplant cyclophosphamide treatment, wherein the stem cell transplant is an allogeneic stem cell transplant.

The present invention is based, at least in part, on the discovery that marrow infiltrating lymphocytes (MILs) from patients treated with post-transplant cyclophosphamide (PTCy) can augment anti-tumor immunity. The present invention addresses the problem of disease relapse following an allogeneic bone marrow transplant with highly tumor specific T cells.

Marrow infiltrating lymphocytes (MILs) from patients treated with post-transplant cyclophosphamide (PTCy) offer the advantage having been depleted of allo-reactive T cells responsible for causing GVHD and being enriched for tumor specific T cells. This approach represents a unique way of delivering a highly tumor specific, low toxicity T cell therapy that would address the treatment of post-allogeneic transplant relapse.

PTCy is an approach to allogeneic bone marrow transplantation developed at Johns Hopkins University that has dramatically reduced the toxicity and mortality of transplantation enabling patients to undergo transplantation with minimal pharmacologic immunosuppression. In addition, the present inventors have previously shown that MILs possess heightened tumor specificity that can be increased upon ex vivo activation and expansion. Utilizing MILs obtained from patients after they have been treated with PTCy offers the unique opportunity to deliver highly tumor specific T cells with minimal toxicity.

In other embodiments, the present invention is applicable to the haplo-identical transplants with post-transplant cyclophosphamide. In other examples, the treatment described herein is applicable to patients undergoing a standard allogeneic transplant even in the absence of post-transplant cyclophosphamide.

The present invention enables patients to be offered a more tumor-specific approach to the standard donor lymphocyte infusion (DLI). As configured, this is a patient-specific product that can be obtained from any patient undergoing some form of an allogeneic hematopoietic stem cell transplant which includes, but is not limited to, HLA-identical bone marrow transplant, HLA-identical peripheral stem cell transplant, matched unrelated donor (MUD) stem cell transplant, haploidentical bone marrow transplant, peripheral stem cell transplant and cord blood transplant. This could also be performed in patients whether the transplant was myeloablative or non-myeloablative.

In particular embodiments, the allogeneic transplant is selected from the group consisting of HLA-identical bone marrow transplant, HLA-identical peripheral stem cell transplant, matched unrelated donor (MUD) stem cell transplant, haploidentical bone marrow transplant, peripheral stem cell transplant and a cord blood transplant. In a specific embodiment, the subject has cancer. In a more specific embodiment, the cancer is a hematological malignancy. In yet another embodiment, the cancer is a leukemia. In a further embodiment, the bone marrow sample is obtained about one month to about a year after transplant. In a specific embodiment, the methods described herein significantly reduce the likelihood of developing GVHD

Further, described herein is a method for treating or preventing post-allogeneic transplant relapse in a subject who has received post-transplant cyclophosphamide treatment comprising the steps of (a) obtaining a bone marrow sample from the subject; (b) expanding the marrow infiltrating lymphocytes (MILs) present in the sample; and (c) administering the MILs to the subject.

Also described herein is a method for treatment of cancer in a subject who has received an allogeneic stem cell transplant that comprises the steps of (a) administering cyclophosphamide to the subject after the transplant; (b) obtaining a bone marrow sample from the subject; (c) expanding the marrow infiltrating lymphocytes (MILs) present in the sample; and (d) administering the MILs to the subject.

Still further, described herein are methods for treating or preventing cancer relapse in a subject who has received a post-stem cell transplant cyclophosphamide treatment comprising the step of administering activated MILs to the subject, wherein the activated MILs are derived from a bone marrow sample taken from the subject following post-transplant cyclophosphamide treatment. In another example, the present specification provides a method for treating or preventing post-allogeneic transplant relapse and reducing the likelihood of developing GVHD in a subject who has received post-transplant cyclophosphamide treatment comprising the steps of (a) obtaining a bone marrow sample from the subject; (b) expanding the marrow infiltrating lymphocytes (MILs) present in the sample; and (c) administering the MILs to the subject.

### BRIEF DESCRIPTION OF THE FIGURES

FIG. 1 is a table showing the effective expansion of allo-MILs from bone marrow samples obtained post-transplant at time frames ranging from 2 months post-transplant to one year.
FIG. 2 is a graph showing allo-MILs of the present invention have the ability to respond to third party antigens with minimal "self' recognition. The allo-MILs demonstrated minimal responsiveness to autologous lymphocytes obtained from the patient pre-transplant but responded robustly to third party, unrelated lymphocytes.
FIG. 3 demonstrates that, upon activation, the vast majority of both the CD4 and CD8 cells possess a central memory phenotype.

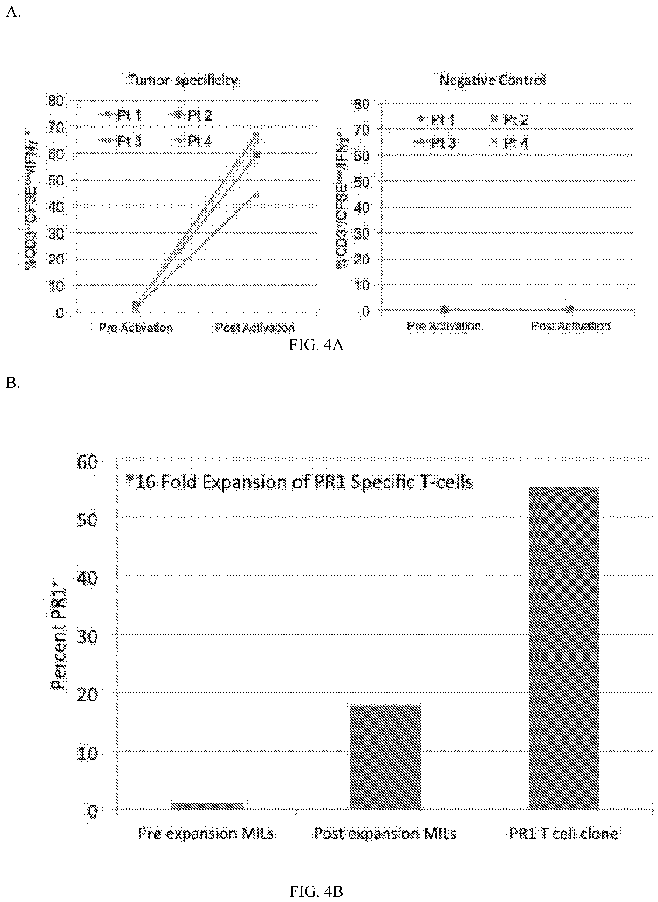

FIG. 4 demonstrates the tumor specificity of the alloMILs. A. Tumor specificity was determined pre- and post-expansion utilizing whole tumor lysate and demonstrating detectable tumor-specificity upon expansion as compared to the negative control using a bladder cancer cell line where no tumor specificity was appreciated. B. The tumor specificity of an HLA-A2+ AML patient in which the percent of PR-1 tumor specific T cells of 16% post-expansion was determined by tetramer analysis.

### DETAILED DESCRIPTION OF THE INVENTION

It is understood that the terminology used herein is used for the purpose of describing particular embodiments only, and is not intended to limit the scope of the present invention. It must be noted that as used herein and in the appended claims, the singular forms "a," "an," and "the" include the plural reference unless the context clearly dictates otherwise. Thus, for example, a reference to a "protein" is a reference to one or more proteins, and includes equivalents thereof known to those skilled in the art and so forth.

Unless defined otherwise, all technical and scientific terms used herein have the same meaning as commonly understood by one of ordinary skill in the art to which this invention belongs. Specific methods, devices, and materials are described, although any methods and materials similar or equivalent to those described herein can be used in the practice or testing of the present invention.

### I. Definitions

The term "amelioration" refers to a reduction of at least one sign and/or symptom of a specific disease or condition. Treatment refers to reduction of at least one sign and/or symptom of a disease or condition to reduce or eliminate at least one sign and/or symptom of the disease or condition, or to prevent progression of the disease or condition. Amelioration and treatment need not be considered separate interventions, but instead can be considered a continuum of therapeutic interventions.

As used herein, "cancer" is understood as a group of diseases or conditions characterized by malignant hyperplasia and/or neoplasia. The present invention is particularly applicable to cancers of the bone marrow or at minimal, involving the bone marrow including, but not limited to, lymphoma and leukemia, which include malignancies derived from hematopoietic (blood-forming) cells. Other types of cancer that may be applicable herein include, but are not limited to: carcinoma, which includes malignant tumors derived from epithelial cells, for example cancers of the breast, prostate, lung and colon; sarcoma which includes malignant tumors derived from connective tissue, or mesenchymal cells; germ cell tumors, which includes tumors derived from totipotent cells, most often found in the testicle and ovary in adults; and in fetuses, babies, and young children most often found on the body midline, particularly at the tip of the tailbone; and blastic tumor or blastoma which includes tumors which resembles an immature or embryonic tissue. Many of these tumors are most common in children.

As used herein, "changed as compared to a control reference sample" is understood as having a level or activity of an analyte, or in a whole organism change of physical characteristics or signs or symptoms of a disease, to be detected at a level that is statistically different than a sample from a normal, untreated, or control sample. Methods to select and test control samples are within the ability of those in the art. Control samples typically include a cell or an animal of the same type that has not been contacted with an active agent or been subjected to a particular treatment, and has optionally been contacted with a carrier or subjected to a sham treatment. Control samples also include a cell or an animal not subjected to an agent or treatment to induce a specific disease or condition.

As used herein, "compound" or "pharmaceutical compound" of the invention and the like include activated MILs prepared by the methods of the specification. The cells can be administered alone or in conjunction with other pharmaceutical agents and compositions, either pharmaceutically active or carrier agents and compositions.

"Contacting a cell" is understood herein as providing an agent to a cell, in culture or in an animal, such that the agent can interact with the surface of the cell, potentially be taken up by the cell, and have an effect on the cell. The agent can be delivered to the cell directly (e.g., by addition of the agent to culture medium or by injection into the cell or tissue of interest), or by delivery to the organism by an enteral or parenteral route of administration for delivery to the cell by circulation, lymphatic, or other means.

As used herein, "detecting", "detection" and the like are understood that an assay performed for identification of a specific analyte in a sample or a change in a subject of at least one sign or symptom of a disease, expression of a protein or gene, including a reporter construct. The amount of analyte detected in the sample or change in a subject can be none or below the level of detection of the assay or method.

The term "detectable label" is understood as a chemical modification, binding agent, or other tag that can be readily observed, preferably in a quantitative manner, such as a fluorescent tag that has specific wavelengths of absorption and emission to allow detection of the compound associated with the detectable label.

The terms "disease" or "condition" are commonly recognized in the art and designate the presence of at least one sign and/or symptom in a subject or patient that are generally recognized as abnormal. Diseases or conditions may be diagnosed and categorized based on pathological changes. Signs may include any objective evidence of a disease such as changes that are evident by physical examination of a patient or the results of diagnostic tests that may include, among others, laboratory tests. Symptoms are subjective evidence of disease or a patient condition, e.g., the patient's perception of an abnormal condition that differs from normal function, sensation, or appearance, which may include, without limitations, physical disabilities, morbidity, pain, and other changes from the normal condition experienced by a subj ect.

The terms "drug", "therapeutic agent", and the like as used herein refer to a chemical entity or biological product, or combination of chemical entities or biological products, administered to a subject to treat or prevent or control a disease or condition. The drug or therapeutic agent can be formulated with one or more pharmaceutically acceptable carriers. Therapeutic agents of the instant invention can be co-administered with other drugs or therapeutic agents. "Co-administering," as used herein refers to the administration with another agent, either at the same time, in the same composition, at alternating times, in separate compositions, or combinations thereof.

As used herein, the terms "effective" and "effectiveness" includes both pharmacological effectiveness and physiological safety. Pharmacological effectiveness refers to the ability of the treatment to result in a desired biological effect in the patient. Physiological safety refers to the level of toxicity, or other adverse physiological effects at the cellular, organ and/or organism level (often referred to as side-effects) resulting from administration of the treatment. On the other hand, the term "ineffective" indicates that a treatment does not provide sufficient pharmacological effect to be therapeutically useful, even in the absence of deleterious effects, at least in the unstratified population. Such a treatment may be ineffective in a subgroup that can be identified by the expression profile or profiles. "Less effective" means that the treatment results in a therapeutically significant lower level of pharmacological effectiveness and/or a therapeutically greater level of adverse physiological effects, e.g., greater liver toxicity.

Thus, in connection with the administration of a drug, a drug which is "effective against" a disease or condition indicates that administration in a clinically appropriate manner results in a beneficial effect for at least a statistically significant fraction of patients, such as an improvement of symptoms, a cure, a reduction in disease signs or symptoms, extension of life, improvement in quality of life, or other effect generally recognized as positive by medical doctors familiar with treating the particular type of disease or condition.

The term "effective amount" refers to a dosage or amount that is sufficient to reduce, halt, or slow tumor progression to result in alleviation, lessening or amelioration of symptoms in a patient or to achieve a desired biological outcome, e.g., slow or stop tumor growth or reduction or disappearance of a tumor.

"Enriched" as used herein is understood as to process so as to add or increase the proportion of a desirable ingredient. As used herein, enrichment is understood as increasing the proportion of a specific cell type from a population of cells, e.g. peripheral blood or bone marrow, for the presence of a specific cell type, particularly immune cells based on the presence or absence of specific cell surface markers. Enrichment also includes cell sorting by methods such as flow cytometery which rely on sorting based on markers.

"Essentially" as used herein is understood as not departing from the fundamental nature or critical element of the method or agent. For example, a culture that is static is understood as being essentially static, that is growth of the culture occurs without stirring or agitation, however, the culture may be moved during the period of cell culture without altering the fundamental nature of a static culture.

As used herein, the term "expanding" is understood as promoting the growth or growing, particularly promoting the growth of a particular cell type within a mixed cell population, e.g., promoting the growth of marrow infiltrating lymphocytes in a mixed population of cells such as fractionated bone marrow from which most of the red blood cells and neutrophils have been removed.

As used herein, "hematological malignancy" is any type of cancer that affects blood, bone marrow, and/or lymph nodes. As the three are intimately connected, a disease affecting one of the three will often affect the others as well. Although lymphoma is technically a disease of the lymph nodes, it often spreads to the bone marrow, affecting the blood. Hematological malignancies include, but are not limited to multiple myeloma, leukemias, and lymphomas.

As used herein, "isolated" or "purified" when used in reference, for example, to a polypeptide or cell means that a naturally occurring polypeptide or cell has been removed from its normal physiological environment (e.g., protein isolated from plasma or tissue; cell isolated from the body) or is synthesized in a non-natural environment (e.g., artificially synthesized in a heterologous system (protein expressed or cultured/expanded cell)). Thus, an "isolated" or "purified" polypeptide can be in a cell-free solution or placed in a different cellular environment (e.g., expressed in a heterologous cell type). The term "purified" does not imply that the polypeptide or cell is the only polypeptide or cell present, but that it is essentially free (about 80-90%, or about 90-95%, up to 99-100% pure) of cellular or organismal material naturally associated with it, and thus is distinguished from naturally occurring polypeptide. "Isolated" when used in reference to a cell means the cell is in culture (i.e., not in an animal), either cell culture or organ culture, of a primary cell or cell line. Cells can be isolated from a normal animal, a transgenic animal, an animal having spontaneously occurring genetic changes, and/or an animal having a genetic and/or induced disease or condition. Isolated cells can be further modified to include reporter constructs or be treated with various stimuli to modulate expression of a gene of interest. A cell can also be isolated from a specific reagent, for example, agents used to treat the cells such as antibody-coated beads. Isolation of the cells from antibody-coated beads includes removal of a sufficient portion of the beads such that the cells are acceptable for administration to a subject, particularly a human subject.

As used herein, "kits" are understood to contain at least a non-standard laboratory reagents or device component for use in the performance of the invention in appropriate packaging with directions for use. The kit can further include any other components required to practice the invention, such as dry powders, concentrated solutions, or ready to use solutions. In some examples, the kit comprises one or more containers that contain reagents for use in the invention; such containers can be boxes, ampules, bottles, vials, tubes, bags, pouches, blister-packs, or other suitable container forms known in the art. Such containers can be made of plastic, glass, laminated paper, metal foil, or other materials suitable for holding reagents.

"Lymphoablation" as used herein is understood as any form of therapy that can include chemotherapy, antibody, or radiation therapy aimed at depleting lymphocytes in vivo but not depleting myeloid elements. Lymphablation can be performed using any of a number of chemotherapy or immunotherapy agents such as those used to deplete lymphocytes in the subject including, but not limited to, busulfan, adriamycin, bexxar, blenoxane, dacarbazine, cyclophosphamide, cytoxan, DTIC, etoposide, matulane, mechlorethamine, mustargen, Rituxan, VCR, orasone, procarbazine, vincristine, and Zevalin® (radioimmunotherapy regimen), radiation or combinations thereof.

As used herein, a "marrow infiltrating lymphocyte" or "MIL" is understood as a T cell present in the bone marrow, particularly a T cell present in the bone marrow of a subject suffering from a hematological malignancy or a metastatic neoplastic disease in which tumor cells are present in the bone marrow. MILs can be activated to become activated MILs ("aMILs") by stimulation with appropriate factors such as anti-CD3 and anti-CD28 antibodies. Fold expansion of aMILS is determined by detection of CD3 cells in the population. The percentage of CD3 is determined on the last day of expansion by staining for CD3. This percentage is multiplied by the total number of cells collected. The total number of CD3 on the last day of expansion is divided by the total number of CD3 on D0 and this is the total fold expansion. Roughly between 10-150 fold expansion is typically achieved using the culture methods provided, for example, in U.S. Patent Publication No. 2011/0223146.

The phrase "pharmaceutically acceptable carrier" is art recognized and includes a pharmaceutically acceptable material, composition or vehicle, suitable for administering compounds used in the methods described herein to subjects, e.g., mammals. The carriers include liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

"Providing," refers to obtaining, by for example, buying or making the, e.g., cells, polypeptide, drug, polynucleotide, probe, and the like. The material provided may be made by any known or later developed biochemical or other technique. Cells can be obtained from a subject to be treated using the methods described herein.

"Regulatory T cells" or "T-regs" are understood as a specialized subpopulation of T cells that act to suppress activation of the immune system and thereby maintain immune system homeostasis and tolerance to self-antigens in a normal subject. In a subject suffering from neoplastic disease, T-regs can suppress an immune response to the tumor. Regulatory T cells come in many forms, including those that express the CD8 transmembrane glycoprotein (CD8+ T cells), those that express CD4, CD25 and Foxp3, and other T cell types that have suppressive function.

A "sample" as used herein refers to a biological material that is isolated from its environment (e.g., blood or tissue from an animal; cells or conditioned media from tissue culture) and is suspected of containing, or known to contain an analyte or other desired material. A sample can also be a partially purified fraction of a tissue or bodily fluid, e.g., from a subject having a specific disease or condition. A reference sample can be a "normal" sample, from a donor not having the disease or condition fluid. A reference sample can also be from an untreated donor or cell culture not treated with an active agent (e.g., no treatment or administration of vehicle only) or not subjected to conditions to induce a disease state. A reference sample can also be taken at a "zero time point" prior to contacting the cell with the agent to be tested.

A "subject" as used herein refers to living organisms. In certain embodiments, the living organism is an animal. In certain preferred embodiments, the subject is a mammal. In certain embodiments, the subject is a domesticated mammal. Examples of subjects include humans, non-human primates, dogs, cats, mice, rats, cows, horses, goats, and sheep. A human subject may also be referred to as a patient. A subject can also be a cadaver.

As used herein, "substantially removed" for example, wherein a specific cell type is substantially removed from a population of cells, is understood is at least 75%, at least 80%, at least 85%, at least 90%, at least 95%, at least 98%, or all detectable cells of a certain type are removed from a population of cells. In a preferred embodiment, cells are subjected to density gradient centrifugation or other methods that do not include labeling of cells for the purpose of sorting one type of cell from anther. However, methods of cell staining and sorting can be used to determine if the desired cell population has been substantially removed from the initial population of cells. In a specific embodiment of the invention, the bone marrow is subjected to density gradient centrifugation to substantially remove red blood cells and neutrophils.

A subject "suffering from or suspected of suffering from" a specific disease, condition, or syndrome has at least one risk factor and/or presents with at least one sign or symptom of the disease, condition, or syndrome such that a competent individual would diagnose or suspect that the subject was suffering from the disease, condition, or syndrome. Methods for identification of subjects suffering from or suspected of suffering from hematological malignancy or metastatic disease with bone marrow involvement is within the ability of those in the art. Methods of identifying specific genetic or lifestyle predispositions to hematological malignancies are well within the ability of those of skill in the art. Subjects suffering from, and suspected of suffering from, a specific disease, condition, or syndrome are not necessarily two distinct groups.

The language "therapeutically effective amount" or a "therapeutically effective dose" of a compound is the amount necessary to or sufficient to provide a detectable improvement in of at least one symptom associated or caused by the state, disorder or disease being treated. The therapeutically effective amount can be administered as a single dose or in multiple doses over time. Two or more compounds can be used together to provide a "therapeutically effective amount" to provide a detectable improvement wherein the same amount of either compound alone would be insufficient to provide a therapeutically effective amount. "Therapeutically effective amount," as used herein refers to an amount of an agent which is effective, upon single or multiple dose administration to the cell or subject, decreasing at least one sign or symptom of the disease or disorder, or prolonging the survivability of the patient with such a disease or disorder beyond that expected in the absence of such treatment.

An agent can be administered to a subject, either alone or in combination with one or more therapeutic agents, as a pharmaceutical composition in mixture with conventional excipient, e.g., pharmaceutically acceptable carrier.

The pharmaceutical agents may be conveniently administered in unit dosage form and may be prepared by any of the methods well known in the pharmaceutical arts, e.g., as described in Remington's Pharmaceutical Sciences (Mack Pub. Co., Easton, Pa., 1980). Formulations for parenteral administration may contain as common excipients such as sterile water or saline, polyalkylene glycols such as polyethylene glycol, oils of vegetable origin, hydrogenated naphthalenes and the like. In particular, biocompatible, biodegradable lactide polymer, lactide/glycolide copolymer, or polyoxyethylene-polyoxypropylene copolymers may be useful excipients to control the release of certain agents.

The compounds of the invention can, for example, be administered by injection, preferably intravascularly, that is intravenously. Methods of administration by injection or infusion can be performed using a pump. The methods herein contemplate administration of an effective amount of compound or compound composition to achieve the desired or stated effect. Typically, the pharmaceutical compositions of this invention will be administered once a day, once a week, every two weeks, once a month, or more or less frequently, depending on the specific needs of the subject to be treated. The specific pharmacokinetic and pharmacodynamic properties of the composition (e.g., persistence and engraftment of the MILs) to be administered will effect dosing. Such administration can be used as a chronic or acute therapy. The amount of active ingredient that may be combined with the carrier materials to produce a single dosage form will vary depending upon the host treated and the particular mode of administration. A typical preparation will contain from about 1% to about 95% active compound (w/w). Alternatively, such preparations contain from about 20% to about 80% active compound.

Cells and/or subjects may be treated and/or contacted with one or more standard cancer therapeutic treatments including, surgery, chemotherapy, radiotherapy, gene therapy, immune therapy, anti-angiogenic therapy, hormonal therapy, bone marrow transplant, or other therapy recommended or proscribed by self or by a health care provider.

The dosage of the activated MILs for use of the present invention will depend on the disease state or condition being treated and other clinical factors such as weight and condition of the human or animal and the route of administration of the compound. It is to be understood that the present invention has application for both human and veterinary use. Single as well as multiple administrations are contemplated for the activated MILs for use according to the present invention. These can be given either simultaneously or over an extended period of time.

Doses used herein in animal experiments correspond to doses of about 1x10⁶/kg to about 2.0x10⁸/kg of MILs. In more specific embodiments, doses can correspond to about 30x10⁷/kg to about 1.0x10⁸/kg of MILs. In specific embodiments, doses can correspond to about 3.3x10⁷/kg, about 8.3x10⁷/kg, and about 1.7x10⁸g of activated MILs. It is understood that any dose within the range of about 1x10⁷/kg to about 5x10⁸/kg, or about 3.3x10⁷/kg to about 1.7x10⁸/kg activated aMILs would be useful in the present invention. It is further understood that dosages may be higher or lower, and that the specific dosage may be altered based on the amount of cells present, the specific disease to be treated, the severity of the disease to be treated, and other considerations known to those of skill in the art.

Lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the disease, condition or symptoms, the patient's disposition to the disease, condition or symptoms, and the judgment of the treating physician.

Upon improvement of a patient's condition, a maintenance dose of a compound, composition or combination of this invention may be administered, if necessary. Subsequently, the dosage or frequency of administration, or both, may be reduced, as a function of the symptoms, to a level at which the improved condition is retained. Patients may, however, require intermittent treatment on a long-term basis upon any recurrence of one or more signs or symptoms of cancer.

Formulations suitable for parenteral administration include aqueous and non-aqueous sterile injection solutions which may contain anti-oxidants, buffers, bacteriostatics, and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampoules and vials, and may be stored in a freeze-dried (lyophilized) condition requiring only the addition of the sterile liquid carrier, for example, water for injections, immediately prior to use. Methods to prepare the MILs of the invention for administration to a subject, typically by intravascular administration, are well within the ability of those of skill in the art. Extemporaneous injection solutions and suspensions may be prepared from sterile powders, granules and tablets of the kind previously described.

### II. Allo-MILs from Post-Transplant Patients Treating with Post-Transplant Cyclophosphamide Augment Anti-Tumor Immunity

The therapeutic benefit of an allogeneic bone marrow transplantation (alloBMT) is the immune-mediated graft vs. tumor effect. This is due to the T cell mediated anti-tumor effect imparted by the donor T cells. Evidence for this is the increased relapse rate in T cell depleted allogeneic transplants. Despite this therapeutic benefit, many patients will eventually relapse with their disease. To date, the standard accepted practice is the use of donor-derived lymphocyte infusions. This approach can induce remissions in a limited number of patients but is also associated with a significantly increased graft vs. host disease (GVHD). Furthermore, the more HLA-disparate donor-host differences, the greater the risk of GVHD. Two recent advances offer the possibility of significantly altering the therapeutic outcomes for these patients. First, the use of post-transplant cyclophosphamide (PTCy) has dramatically reduced the incidence of GVHD enabling us to perform allogeneic transplant across HLA-disparate barriers with no increased incidence of GVHD. Furthermore, the efficacy in reducing GVHD has enabled patients to forgo the need of persistent pharmacologic immunosuppression or receive only minimal periods of immunosuppression. Second, the present inventors have demonstrated the ability to obtain marrow infiltrating lymphocytes (MILs) from patients with hematologic malignancies and to activate and expand them ex vivo with resultant heightened tumor specificity. *See* U.S. Patent Publication No. 2011/0223146. As described herein, the present inventors have developed a more tumor specific approach for donor lymphocyte infusions.

Post-transplant cyclophosphamide works as prophylaxis for GVHD by effectively eliminating highly proliferative T cells which in the early post-transplant period are primarily the allo-reactive T cells. This results in delayed T cell immune reconstitution. However, the T cells that eventually traffic to the bone marrow are tumor specific - albeit at a lower precursor frequency. The present inventors have demonstrated that post-transplant MILs from patients that have received PTCy have heightened tumor specificity and minimal allo-reactivity which can subsequently be expanded to increase the efficacy and safety of allogeneic MILs.

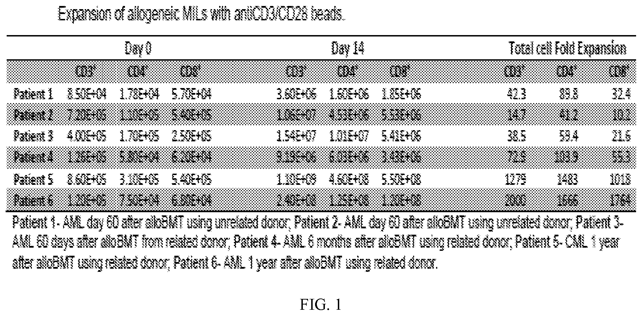

As shown in FIG. 1, the present inventors have been able to effectively expand allo-MILs from bone marrow samples obtained post-transplant at time frames ranging from 2 months post-transplant to further out. This data underscores the technical feasibility of this approach which is critical considering the significant immune suppression that accompanies the early post-transplant period. It is the absence of pharmacologic immune suppression made possible with the use of PTCy that enables the growth of these allo-MILs and to make such a therapeutic approach feasible.

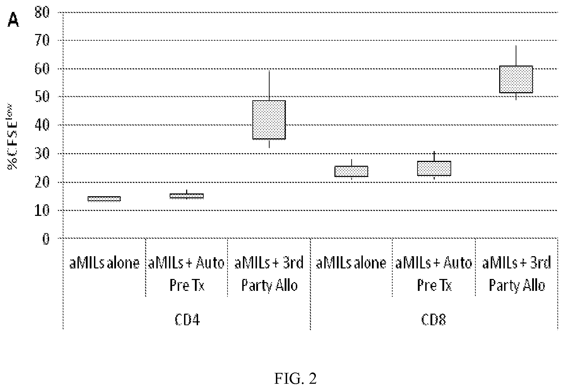

In addition to the technical feasibility of growing allo-MILs, critical to the success of this approach is the ability to demonstrate their ability to respond to third party antigens with minimal "self' recognition. To examine this, activated allo-MILs were labeled with CFSE and their proliferative response was determined by CFSE dilution. As shown in FIG. 2, allo-MILs demonstrated minimal responsiveness to autologous lymphocytes obtained from the patient pre-transplant but responded robustly to third party, unrelated lymphocytes. Taken together, the absence of reactivity to self antigens but robust responsiveness to third party antigens supports the hypothesis that the depletion of allo-reactive, host-specific T cells by the administration of PT-Cy results in a population of MILs potentially able to be utilized clinically with a low likelihood of generating GVHD but with the ability to impart a anti-tumor effect.

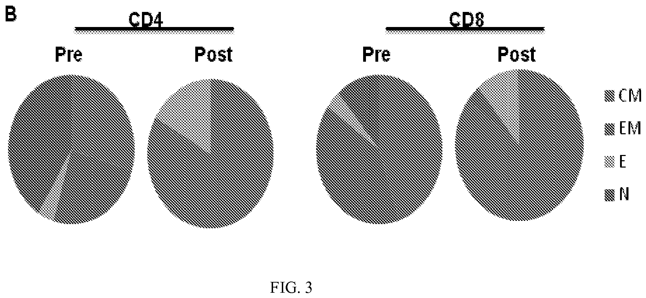

The MILs were subsequently characterized based on a series of functional properties. In light of the work by Berger et al. demonstrating the ability of central memory T cells to impart long-term immunity, the present inventors sought to determine their functional status both pre- and post-activation. Berger et al., 118(1) J. CLIN. INVEST. 294-305 (2008). As shown in FIG. 3, the present inventors demonstrate that upon activation the vast majority of both the CD4 and CD8 cells possess a central memory phenotype. These findings are consistent with previously published data demonstrating the ability of the bone marrow to serve as a reservoir of memory T cells (Feuerer et al., 7(4) NAT. MED. 452-58 (2001) and underscore the unique properties of the bone marrow that justify the rationale for the use of these cells therapeutically purposes.

The major reason for pursuing this approach of PTCy allogeneic MILs is based on the need to increase the therapeutic efficacy of donor lymphocyte infusions (DLI) in patients post-transplant. PTCy has effectively been able to minimize the incidence of GVHD However, relapse post allogeneic transplant remains an area with a significant unmet need. The present inventors hypothesize that this combination of PTCy and subsequent expansion of resident allo-MILs offers a significant therapeutic improvement over the current standard approach. To demonstrate the tumor specificity, two different assays were performed. For the patients in which a tumor specific antigen is not known such as ALL or myeloma, an assay developed by the present inventors was utilized which takes advantage of the endogenous antigen presenting cells (APCs) present in the bone marrow capable of processing and presenting antigens with the appropriate HLA restriction. The present inventors have previously utilized this approach to demonstrate tumor specific immunity in other studies. Noonan et al., 18(5) CLIN. CANCER RES. 1426-34 (2012).

Utilizing this approach, the bone marrow was pulsed with lysate from allogeneic tumor cell lines and demonstrated a significant increase in the tumor specificity of the activated allo-MILs as measured by IPNγ production of the divided, CFSE^{low} cells which went from no detectable IPNγ-pre-activation to IPNγ-production in approximately 50% in the CFSE^{low} cells post-activation. The present inventors were also able to show in one HLA-A2⁺ patient with AML that the antigen specific response to the PR1 peptide of proteinase-3, a tumor associated antigen, increased from 2% to 18% of the entire T cell repertoire.

Patients that have undergone an allogeneic stem cell transplant and are ideally on no pharmacologic immunosuppression undergo a bone marrow aspiration up to 200ml. The bone marrow can be collected in heparinized syringes. The cells are then Ficolled (or undergo an equivalent procedure) to reduce the red cell volume. The percentage of CD3⁺ cells is established to determine the appropriate number of beads that are added. At the time of expansion, the cells (either fresh or thawed) are placed in a tissue culture container and the appropriate number of anti-CD3/CD28 beads is added in addition to IL-2 200IU/ml and 2% human serum. The cells are grown in a static culture until visible T cell blasts can be appreciated at which time the cells are transferred to a dynamic culture system such as the WAVE bioreactor. Cells are perfused with tissue culture medium containing the same concentration of IL-2 and human serum until the appropriate fold-expansion or total T cell number is achieved. The magnetic beads are then depleted by passaging over a magnet. The cells are then concentrated and either infused fresh or frozen and stored in liquid nitrogen to be infused at a later date.

Taken together, the present inventors believe that this is a novel, highly tumor specific approach of adoptive allogeneic T cell therapy aimed at improving the therapeutic efficacy of donor lymphocyte infusions. This approach can be utilized in both HLA-identical as well as haploidentical transplants.

## Claims

1. Activated marrow infiltrating lymphocytes (MILs) for use in a method for treating or preventing cancer relapse in a subject who has received a post-stem cell transplant cyclophosphamide treatment, the method comprising the step of administering the activated MILs to the subject, wherein the activated MILs are derived from a bone marrow sample taken from the subject following the post-stem cell transplant cyclophosphamide treatment, wherein the stem cell transplant is an allogeneic stem cell transplant.

2. The activated MILs for use according to claim 1, wherein the method significantly reduces the likelihood of developing GVHD.

3. The activated MILs for use according to claim 1 or claim 2, wherein the allogeneic stem cell transplant is selected from the group consisting of HLA-identical bone marrow transplant, HLA-identical peripheral stem cell transplant, matched unrelated donor (MUD) stem cell transplant, haploidentical bone marrow transplant, haploidentical peripheral stem cell transplant and a cord blood transplant.

4. The activated MILs for use according to any preceding claim, wherein the cancer is a hematological malignancy.

5. The activated MILs for use according to claim 4, wherein the cancer is a leukemia.

6. The activated MILs for use according to any preceding claim, wherein the bone marrow sample is obtained about one month to about one year after the transplant.

7. The activated MILs for use according to any preceding claim wherein the activated MILs are comprised in a composition.

## Patentansprüche

1. Aktivierte mark-infiltrierende Lymphozyten (MILs) zur Verwendung in einem Verfahren zum Behandeln oder Verhindern eines Krebsrezidivs in einer Person, die eine Cyclophosphamid-Behandlung nach Stammzellentransplantation erhalten hat, wobei das Verfahren den Schritt eines Verabreichens der aktivierten MILs an die Person aufweist, wobei die aktivierten MILs aus einer Knochenmarkprobe stammen, die der Person nach der Cyclophosphamid-Behandlung nach Stammzellentransplantation entnommen wurde, wobei das Stammzellentransplantat ein allogenes Stammzellentransplantat ist.

2. Aktivierte MILs zur Verwendung nach Anspruch 1, wobei das Verfahren die Wahrscheinlichkeit eines Entwickelns einer GvHD signifikant reduziert.

3. Aktivierte MILs zur Verwendung nach Anspruch 1 oder Anspruch 2, wobei das allogene Stammzellentransplantat ausgewählt ist aus der Gruppe bestehend aus HLA-identischem Knochenmarktransplantat, HLA-identischem peripheren Stammzellentransplantat, Matched-Unrelated Donor (MUD) - Stammzellentransplantat, haploidentischem Knochenmarktransplantat, haploidentischem peripheren Stammzelltransplantat und Nabelschnurbluttransplantat.

4. Aktivierte MILs zur Verwendung nach einem der vorhergehenden Ansprüche, wobei der Krebs eine hämatologische Malignität ist.

5. Aktivierte MILs zur Verwendung nach Anspruch 4, wobei der Krebs eine Leukämie ist.

6. Aktivierte MILs zur Verwendung nach einem der vorhergehenden Ansprüche, wobei die Knochenmarkprobe etwa einen Monat bis etwa ein Jahr nach der Transplantation eingeholt wird.

7. Aktivierte MILs zur Verwendung nach einem der vorhergehenden Ansprüche, wobei die aktivierten MILs in einer Mischung enthalten sind.

## Revendications

1. Lymphocytes infiltrant la moelle osseuse (MILs) activés pour une utilisation dans un procédé de traitement ou de prévention de la rechute du cancer chez un sujet qui a reçu un traitement par cyclophosphamide après une greffe de cellules souches, où le procédé comprend l'étape d'administrer des MILs activés au sujet, les MILs activés étant dérivés d'un échantillon de moelle osseuse prélevé sur le sujet à la suite du traitement par cyclophosphamide après une greffe de cellules souches, la greffe de cellules souches étant une greffe de cellules souches allogéniques.

2. MILs activés pour une utilisation selon la revendication 1, où le procédé réduit considérablement la probabilité de développer une GVHD

3. MILs activés pour une utilisation selon la revendication 1 ou 2, où la greffe de cellules souches allogéniques est choisie dans le groupe consistant en une greffe de moelle osseuse HLA-identique, une greffe de cellules souches périphériques HLA-identique, une greffe de cellules souches de donneur non apparenté compatible (MUD), une greffe de moelle osseuse haploidentique, une greffe de cellules souches périphériques haploidentique et une greffe de sang de cordon.

4. MILs activés pour une utilisation selon l'une quelconque des revendications précédentes, où le cancer est une malignité hématologique.

5. MILs activés pour une utilisation selon la revendication 4, où le cancer est une leucémie.

6. MILs activés pour une utilisation selon l'une quelconque des revendications précédentes, où l'échantillon de moelle osseuse est obtenu environ un mois à environ un an après la transplantation.

7. MILs activés pour une utilisation selon l'une quelconque des revendications précédentes, où les MILs activés sont compris dans une composition.
